# EUROPEAN PATENT APPLICATION

(11) **EP 4 541 824 A1**
(43) Date of publication of application: **23.04.2025**
(21) Application number: 23834745.4
(22) Date of filing: 30.06.2023
(51) Int. Cl.: C07K 19/00, C07K 14/755, C12N 15/85

(54) **LINKER PEPTIDE, FVIII PROTEIN CONTAINING LINKER PEPTIDE OR VARIANT THEREOF, AND USE THEREOF**

(30) Priority: 07.07.2022 CN 202210823387
(71) Applicant: Shenzhen Syno Minicircle Biotechnology Co., Ltd., Shenzhen, Guangdong 518107 (CN)
(72) Inventor: CHEN, Ping, Shenzhen, Guangdong 518107 (CN); LIU, Jie, Shenzhen, Guangdong 518107 (CN); HOU, Xiaohu, Shenzhen, Guangdong 518107 (CN); CHEN, Guochuang, Shenzhen, Guangdong 518107 (CN); CHEN, Zhiying, Shenzhen, Guangdong 518107 (CN)
(74) Representative: Frick, Robert
(86) International application number: PCT/CN2023/104477
(87) International publication number: WO 2024/007978

(57) **Abstract**

The invention relates to a linker peptide, a Factor VIII protein containing the linker peptide or its variant thereof, and their use thereof, falling within the field of gene therapy for genetic defects. The linker peptide can enhance the expression level, activity, and therapeutic effect of Factor VIII protein or its variant with a microcircular DNA vector. Additionally, it exhibits favorable therapeutic effects upon secondary dosing, as well as advantages such as good safety and long-term expression in vivo.

## Description

### FIELD OF THE INVENTION

The present invention relates to the field of genetic defect treatment drugs, specifically a linker peptide, an FVll protein containing the linker peptide or a variantthereof, and the use thereof.

### BACKGROUND OF THE INVENTION

Hemophilia is a group of X-chromosome-linked monogenic deficiency inherited diseases characterized by coagulation disorders due to coagulation factor deficiencies. Among them, Hemophilia A, which is caused by a lack of FVIII encoded by the F8 gene, is the most common form, affecting up to 80-85% of patients. Currently, the standard treatment for Hemophilia A is replacement therapy, which involves supplementing exogenous FVIII protein, including plasma-derived FVIII (pdFVIII) and recombinant FVIII (rFVIII). pdFVIII is extracted from normal human plasma, and its supply is limited, with risks of blood-borne virus transmission. rFVIII is produced through expression and purification in mammalian cell lines in vitro, which is not limited by blood supply and can effectively reduce the risk of blood-borne virus infection, but it requires highly sophisticated production processes.

The FVIII protein has a short half-life in vivo (average of 12 hours), necessitating lifelong administration and frequent injections (2-3, or even 4 intravenous injections per week) for patients undergoing replacement therapy, resulting in high costs and inconvenience. Conventional long-acting modifications such as Fc fusion (Drug Des Devel Ther 2014, 8:365-371) and PEG modification (Haemophilia 2019, 25:773-781) can extend its half-life to about 19 hours, but only to a limited extent (an increase of 50-60%), and do not significantly reduce the frequency of dosing. Sanofi and Sobi have jointly developed a more long-acting novel FVIII fusion protein (BIVV001; FVIIIFc-vWF-XTEN), which further extends the half-life to 38-44 hours by fusing the Fc fragment of an antibody and the D'D3 domain of the vWF factor, reducing the dosing frequency to once a week (N Engl J Med. 2020, 383:1018-1027). Although these long-acting modifications of protein molecules can extend the half-life to varying degrees and improve the patient's medication experience to some extent, they cannot fundamentally change the treatment mode of "lifelong dosing and repeated injections."

Conversely, FVIII gene therapy aims to correct the genetic defects of Hemophilia A patients at the genetic level, potentially achieving functional "cure" of the disease. Currently, AAV-hFVIII (adeno-associated virus vector for liver-targeted delivery of the normal human FVIII gene) is widely regarded as the "best" option for gene therapy for Hemophilia A. However, existing liver-targeted AAV-FVIII gene therapy has significant limitations (Hemasphere 2021, 5:e540):
i) AAV-hFVIII is only suitable for adult patients aged 18 and above. The livers of children are in rapid growth and development, with hepatocytes dividing actively, making them unsuitable for this therapy (substantially increased risk of gene integration; unintegrated target genes are easily lost during cell division).
ii) AAV is a common virus in humans with a high infection rate. Therefore, many patients have pre-existing AAV neutralizing antibodies, rendering this therapy unusable for these patient groups.
iii) Repeated dosing is not possible. AAV-hFVIII is not lifelong effective, and its expression level may fall below the therapeutic level after several years. At this point, redosing is required to restore therapeutic levels. However, the AAV capsid is highly immunogenic, and virus-specific immune responses are easily induced after the first dose, rendering subsequent injections ineffective.
iv) The hFVIII coding gene is large, exceeding the capacity limit of AAV vectors, making AAV-hFVIII packaging and production difficult. Additionally, AAV therapy has other safety concerns such as random gene integration.

The expression of hFVIII (human Factor VIII) is quite challenging, representing a common and critical technical challenge for both recombinant FVIII production (in vitro expression) and FVIII gene therapy (in vivo expression). The expression level of hFVIII is only 1% to 0.1% of that of ordinary proteins with comparable molecular weights (Hum Gene Ther 1993, 4:259-272; Blood 2004, 103:3412-3419). Therefore, modifying the hFVIII molecule to improve its expression level has become a focus of effort in related fields. FVIII consists of six domains: A1-A2-B-A3-C1-C2, with the B region being 908 aa long, accounting for about 40% of the full length (2332 aa). Deleting the B region does not affect its coagulation function (PNAS 1986, 83:5939-5942) and can significantly increase mRNA (17-fold) and protein product (30%) levels (Blood 2004, 103:3412-3419; Blood Coagul Fibrinolysis 1997, 8 Suppl 2:S3-14). Therefore, deletion of the B region has become a widely adopted modification method; in fact, multiple B-domain-deleted FVIII drugs (BDD-FVIII) have been clinically used on a large scale for many years, such as Xyntha produced by Pfizer. The earliest BDD-FVIII almost completely deleted the B region, retaining only a linker consisting of 4 amino acids from the N-terminus and 10 amino acids from the C-terminus of the natural FVIII B region (a total of 14 aa) to connect A1-A2 with A3-C1-C2 (this linker is called the SQ linker; the sequence is SFSQNPPVLKRHQR). For convenience, BDD-FVIII containing the SQ linker is denoted as BDD-FVIII-SQ. We attempted to study the feasibility of using a mini-circle DNA vector for in vivo expression of therapeutic levels of BDD-FVIII-SQ; however, experiments found that the BDD-FVIII-SQ vector could only express very weakly in vivo (less than 1% of normal levels), failing to achieve therapeutic effects. Therapeutic levels have three tiers: i) exceeding 1% for preliminary efficacy, alleviating severe hemophilia to moderate; ii) exceeding 5% for significant efficacy, significantly alleviating moderate to severe hemophilia to mild; iii) exceeding 50% for functional "cure," fully restoring normal coagulation function.

The B-region linker connects A1-A2 with A3-C1-C2, and its sequence has an important impact on the expression level of the BDD-FVIII protein. Miao et al. (Blood 2004, 103:3412-3419) reported that retaining the first 226 amino acids (226aa/N6) of the N-terminus of the B region results in a 4-fold higher expression level than almost completely deleting the B region (wild-type SQ linker) when detected by ELISA. McIntosh et al. (Blood 2013, 121:3335-3344; Patent: WO 2013/186563) further replaced the 226aalN6 with a 31 aa-long v3 linker (sequence: SFSQNATNVSNNSNTSNDSNVSPPVLKRHQR) on this basis and found that it could further increase the expression level of the target protein (by about 50%). The present invention denotes BDD-FVIII containing the v3 linker as BDD-FVIII-v3. Currently, AAV gene therapy based on BDD-FVIII-v3 has initiated Phase I clinical trials (NCT03001830). Novo Nordisk (Denmark) has developed another BDD-FVIII molecule (N8) with better expression than BDD-FVIII-SQ, containing a 21 aa-long linker (sequence: SFSQNSRHPSQNPPVLKRHQR) (Haemophilia 2010, 16:349-359; Patent: WO2006103298); the present invention denotes this molecule as BDD-FVIII-N8. However, BDD-FVIII-v3 and BDD-FVIII-N8 still have issues such as low expression levels and poor therapeutic effects.

Therefore, there is still an urgent need for a drug for treating diseases related to Factor VIII deficiency that has high expression levels, allows for secondary dosing, is simple to prepare, has good therapeutic effects, and can be expressed long-term in vivo.

### Summary of the Invention

In order to solve the above problems, the technical solutions provided by the present disclosure are as follows.

In a first aspect, a linker peptide is provided. The recombinant FVIII protein or the variant thereof using the linker peptide provided by the present disclosure has the advantages such as high expression level, good effect in secondary administration, simple preparation, and good therapeutic effect. The minicircle DNA of the recombinant FVIII protein or the variant thereof using the linker peptide provided by the present disclosure has the advantages such as high expression level, good effect in secondary administration, simple preparation and, good therapeutic effect, and also solves the problems of difficulty in production (difficulty in packaging), limitation in application range (failure to be used in children, failure to perform secondary administration), and safety risk (carcinogenesis due to random integration) of AAV.hf8, that is, the advantages such as simple production, wide application range (capability of being used in children, good efficacy in secondary administration), good safety, and long-term expression in vivo are provided.

In a second aspect, a nucleotide sequence is provided. The nucleotide sequence can encode the linker peptide of the first aspect.

In a third aspect, the use of the linker peptide of the first aspect or the nucleotide sequence of the second aspect is provided.

In a fourth aspect, a recombinant FVIII protein or a variant thereof is provided. The recombinant FVIII protein or the variant thereof has the advantages such as high expression level, good effect in secondary administration, simple preparation, and good therapeutic effect.

In a fifth aspect, a nucleotide is provided.

In a sixth aspect, a recombinant gene vector is provided.

In a seventh aspect, a parental plasmid for use in the production of a minicircle DNA is provided.

In an eighth aspect, a method for preparing a minicircle DNA is provided.

In a ninth aspect, a minicircle DNA obtained according to the preparation method of the eighth aspect is provided. The minicircle DNA has the advantages such as high expression level, good effect in secondary administration, simple preparation, and good therapeutic effect, and solves the problems of difficulty in production (difficulty in packaging), limitation in application range (failure to be used in children, failure to perform secondary administration), and safety risk (carcinogenesis due to random integration) of AAV.hf8, that is, the advantages such as simple production, wide application range (capability of being used in children, good efficacy in secondary administration), good safety, and long-term expression in vivo are provided.

In a tenth aspect, a host cell comprising a nucleotide sequence encoding the recombinant FVIII protein or the variant thereof of the fourth aspect, the nucleotide sequence of the fifth aspect, the recombinant gene vector of the sixth aspect or the minicircle DNA of the ninth aspect is provided.

In an eleventh aspect, a pharmaceutical composition is provided.

In a twelfth aspect, the use of the recombinant FVIII protein or the variant thereof of the fourth aspect, the nucleotide sequence of the fifth aspect, the recombinant gene vector of the sixth aspect, the parental plasmid of the seventh aspect, the minicircle DNA obtained according to the preparation method of the eighth aspect, the minicircle DNA of the ninth aspect, the host cell of the tenth aspect or the pharmaceutical composition of the eleventh aspect for the preparation of a drug for treating a disease is provided.

### BRIEF SUMMARY OF THE DRAWINGS

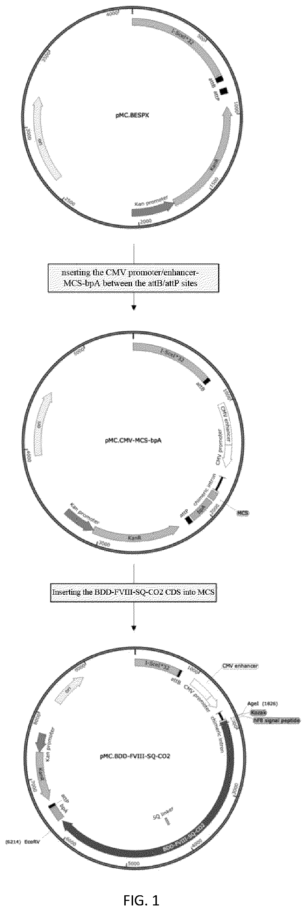

FIG. 1 is a schematic diagram of the construction process of the minicircle DNA parent plasmid pMC.BDD-FVIII-SQ-CO2 in Example 1.

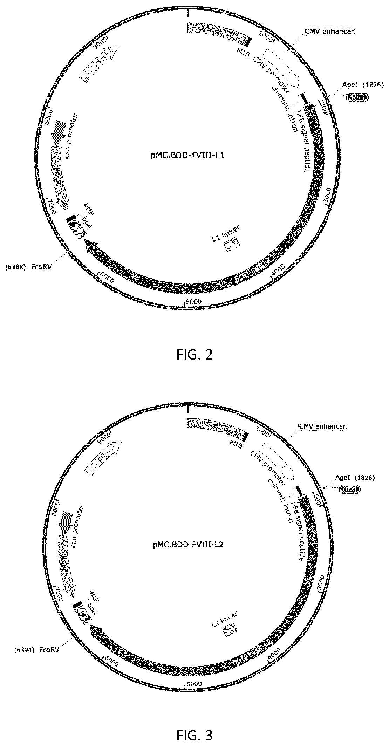

FIG. 2 is a schematic structural diagram of the minicircle DNA parent plasmid pMC.BDD-FVIII-L1 in Example 1.
FIG. 3 is a schematic structural diagram of the minicircle DNA parent plasmid pMC.BDD-FVIII-L2 in Example 1.

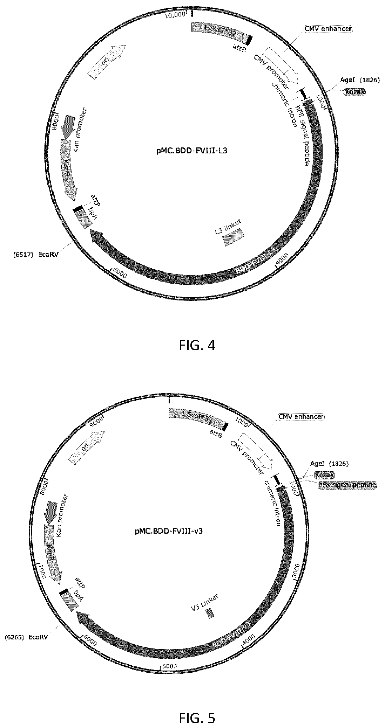

FIG. 4 is a schematic structural diagram of the minicircle DNA parent plasmid pMC.BDD-FVIII-L3 in Example 1.
FIG. 5 is a schematic structural diagram of the minicircle DNA parent plasmid pMC.BDD-FVIII-v3 in Example 1.
FIG. 6 is a schematic structural diagram of the minicircle DNA parent plasmid pMC.BDD-FVIII-N8 in Example 1.
FIG. 7 is a Western blot detection diagram of MC.BDD-FVIII-SQ-CO2 in Example 4; wherein the lane M is a protein marker; the lane 1 is a negative control sample (blank control without transfection of MC vector); the lane 2 is MC.BDD-FVIII-SQ-CO2 cell culture supernatant; and the lane 3 is a positive control sample (hFVIII plasma extract, available from Guizhou Taibang Biological Products Co., Ltd.).
FIG. 8 is a statistical diagram of the results of measuring the *in vitro* cell expression levels of the target proteins of 293T cells transfected by MC.BDD-FVIII-SQ-WT, MC.BDD-FVIII-SQ-CO1 and MC.BDD-FVIII-SQ-CO2 in Example 5 using ELISA.
FIG. 9 is a statistical diagram of the therapeutic effect of MC.BDD-FVIII-SQ-CO2 on mice with hemophilia A and F8 gene knockout (F8-KO) in Comparative Example 1, wherein m1/2mm means that 1 month after the injection, a tail-cutting experiment is performed at a site where the tail has a diameter of 2 mm, and m2/2.5mm means that 2 months after the injection, a tail-cutting experiment is performed at a site where the tail has a diameter of 2.5 mm .
FIG. 10 is a statistical diagram of the results of measuring the *in vitro* cell expression levels of the target proteins of 293T cells transfected by MC.BDD-FVIII-L1, MC.BDD-FVIII-L2, and other FVIII minicircle DNA vectors in Example 6.
FIG. 11 is a statistical diagram of the expression levels of MC.BDD-FVIII-L1 and MC.BDD-FVIII-L2 in normal mice in Example 8.
FIG. 12 is a statistical diagram of the *in vivo* expression level of MC.BDD-FVIII-L1 in beagle dogs in Example 8.
FIG. 13 is a statistical diagram of the expression level of MC.BDD-FVIII-L1 in mice with hemophilia A (F8-KO mice) in Example 9.
FIG. 14 is a statistical diagram of blood loss in the tail-cutting experiments of the F8-KO mice in the blank control group (PBS), the F8-KO mice in the MC.BDD-FVIII-L1 minicircle DNA treatment group (MC) and the wild-type normal mice (WT) in Example 9.
FIG. 15 is a statistical diagram of blood loss in the tail-cutting experiments of the F8-KO mice in the blank control group (PBS), the F8-KO mice in the MC.BDD-FVIII-L2 minicircle DNA treatment group (MC) and the wild-type normal mice (WT) in Example 9.
FIG. 16 is a statistical diagram of the *in vivo* expression level of MC.BDD-FVIII-CTP-Fc in Example 10.
FIG. 17 is a statistical diagram of the results of measuring the *in vitro* cell expression levels of the target proteins of 293T cells transfected by MC.BDD-FVIII-L1-X5 and MC.BDD-FVIII-L1 in Example 10 using ELISA.
FIG. 18 is a statistical diagram of the results of target protein expression level of MC.BDD-FVII-L1-X5 and MC.BDD-FVIII-L1 in the plasma samples in the *in vivo* experiments in mice in Example 10.
FIG. 19 is a statistical diagram of the therapeutic effects of secondary administration of MC.BDD-FVIII-L1 and MC.BDD-FVIII-L2 in Example 11.
FIG. 20 is a graph showing the long-term expression of MC.BDD-FVIII-L1 minicircle DNA and MC.BDD-FVIII-L2 minicircle DNA in mice in Example 12, respectively.
FIG. 21 is a diagram showing the pathological section results of beagle dogs after the injection with MC.BDD-FVIII-L1 minicircle DNA in Example 13.

### Detailed Description of the Invention

In order to solve the above problems, the technical solutions provided by the present disclosure are as follows.

In a first aspect, a linker peptide is provided.

A linker peptide having an amino acid sequence of SEQ ID NO. 1 or SEQ ID NO. 2, or having an amino acid sequence having at least 80%-99% identity to any one of the nucleotide sequences or having at least a portion of either of the sequences. The recombinant FVIII protein or the variant thereof using the linker peptide provided by the first aspect of the present disclosure has the advantages such as high expression level, good effect in secondary administration, simple preparation, and good therapeutic effect. The minicircle DNA of the recombinant FVIII protein or the variant thereof using the linker peptide provided by the first aspect of the present disclosure has the advantages such as high expression level, good effect in secondary administration, simple preparation, and good therapeutic effect, and also solves the problems of difficulty in production (difficulty in packaging), limitation in application range (failure to be used in children, failure to perform secondary administration), and safety risk (carcinogenesis due to random integration) of AAV.hf8, that is, the advantages such as simple production, wide application range (capability of being used in children, good efficacy in secondary administration), good safety, and long-term expression in vivo are provided.

In some embodiments, the nucleotide sequence encoding the linker peptide having an amino acid sequence of SEQ ID NO. 1 (abbreviated as L1 linker in the present disclosure) may include SEQ ID NO. 3 or a codon-optimized sequence thereof.

In some embodiments, the nucleotide sequence encoding the linker peptide having an amino acid sequence of SEQ ID NO. 2 (abbreviated as L2 linker in the present disclosure) may include SEQ ID NO. 4 or a codon-optimized sequence thereof.

In a second aspect, a nucleotide sequence is provided.

A nucleotide sequence encoding the linker peptide of the first aspect.

In some embodiments, the nucleotide sequence includes SEQ ID NO. 3 or SEQ ID NO. 4, or a codon-optimized sequence of either of the nucleotide sequences.

In some embodiments, the nucleotide sequence of SEQ ID NO. 3 or a codon-optimized sequence thereof may be used to encode a linker peptide having an amino acid sequence of SEQ ID NO. 1.

In some embodiments, the nucleotide sequence of SEQ ID NO. 4 or a codon-optimized sequence thereof may be used to encode a linker peptide having an amino acid sequence of SEQ ID NO. 2.

In a third aspect, the use of the linker peptide of the first aspect or the nucleotide sequence of the second aspect is provided.

Use of the linker peptide of the first aspect or the nucleotide sequence of the second aspect for the construction of a recombinant FVIII protein or a variant thereof.

Use of the nucleotide sequence of the second aspect for the construction of a nucleotide sequence of a recombinant FVIII protein or a variant thereof.

In a fourth aspect, a recombinant FVIII protein or a variant thereof is provided.

An FVIII protein or a variant thereof, wherein the linker peptide of the FVIII protein or the variant thereof is selected from the linker peptide of the first aspect, or the nucleotide sequence of the linker peptide of the FVIII protein or the variant thereof is selected from the nucleotide sequence of the second aspect. The recombinant FVIII protein or the variant thereof provided by the fourth aspect of the present disclosure has the advantages such as high expression level, good effect in secondary administration, simple preparation, and good therapeutic effect.

In some embodiments, the recombinant FVIII protein or the variant thereof has an amino acid sequence of SEQ ID NO. 5, SEQ ID NO. 6, SEQ ID NO. 21, SEQ ID NO. 23, SEQ ID NO. 43, SEQ ID NO. 44, SEQ ID NO. 50, SEQ ID NO. 51, SEQ ID NO. 52 or SEQ ID NO. 53, or has an amino acid sequence having at least 80%-99% identity to any one of the amino acid sequences or has at least a portion of any one of the amino acid sequences.

In some embodiments, the nucleotide sequence encoding the recombinant FVIII protein or the variant thereof having the amino acid sequence of SEQ ID NO. 5 may include SEQ ID NO. 7 or a codon-optimized sequence thereof.

In some embodiments, the nucleotide sequence encoding the recombinant FVIII protein or the variant thereof having the amino acid sequence of SEQ ID NO. 6 may include SEQ ID NO. 8 or a codon-optimized sequence thereof.

In some embodiments, the nucleotide sequence encoding the recombinant FVIII protein or the variant thereof having the amino acid sequence of SEQ ID NO. 21 may include SEQ ID NO. 22 or a codon-optimized sequence thereof.

In some embodiments, the nucleotide sequence encoding the recombinant FVIII protein or the variant thereof having the amino acid sequence of SEQ ID NO. 23 may include SEQ ID NO. 24 or a codon-optimized sequence thereof.

In some embodiments, the nucleotide sequence encoding the recombinant FVIII protein or the variant thereof having the amino acid sequence of SEQ ID NO. 43 may include SEQ ID NO. 38 or a codon-optimized sequence thereof.

In some embodiments, the nucleotide sequence encoding the recombinant FVIII protein or the variant thereof having the amino acid sequence of SEQ ID NO. 44 may include SEQ ID NO. 39 or a codon-optimized sequence thereof.

In some embodiments, the nucleotide sequence encoding the recombinant FVIII protein or the variant thereof having the amino acid sequence of SEQ ID NO. 50 may include SEQ ID NO. 56 or a codon-optimized sequence thereof.

In some embodiments, the nucleotide sequence encoding the recombinant FVIII protein or the variant thereof having the amino acid sequence of SEQ ID NO. 51 may include SEQ ID NO. 60 or a codon-optimized sequence thereof.

In some embodiments, the nucleotide sequence encoding the recombinant FVIII protein or the variant thereof having the amino acid sequence of SEQ ID NO. 52 may include SEQ ID NO. 58 or a codon-optimized sequence thereof.

In some embodiments, the nucleotide sequence encoding the recombinant FVIII protein or the variant thereof having the amino acid sequence of SEQ ID NO. 53 may include SEQ ID NO. 54 or a codon-optimized sequence thereof.

In a fifth aspect, a nucleotide is provided.

A nucleotide sequence encoding the recombinant FVIII protein or the variant thereof of the fourth aspect.

In some embodiments, the nucleotide sequence includes SEQ ID NO. 7, SEQ ID NO. 8, SEQ ID NO. 22, SEQ ID NO. 24, SEQ ID NO. 38, SEQ ID NO. 39, SEQ ID NO. 54, SEQ ID NO. 56, SEQ ID NO. 58 or SEQ ID NO. 60, or a codon-optimized sequence of any one of the nucleotide sequences.

In some embodiments, the nucleotide sequence of SEQ ID NO. 7 may be used to encode the recombinant FVIII protein or the variant thereof having an amino acid sequence of SEQ ID NO. 5.

In some embodiments, the nucleotide sequence of SEQ ID NO. 8 may be used to encode the recombinant FVIII protein or the variant thereof having an amino acid sequence of SEQ ID NO. 6.

In some embodiments, the nucleotide sequence of SEQ ID NO. 22 may be used to encode the recombinant FVIII protein or the variant thereof having an amino acid sequence of SEQ ID NO. 21.

In some embodiments, the nucleotide sequence of SEQ ID NO. 24 may be used to encode the recombinant FVIII protein or the variant thereof having an amino acid sequence of SEQ ID NO. 23.

In some embodiments, the nucleotide sequence of SEQ ID NO.38 may be used to encode the recombinant FVIII protein or the variant thereof having an amino acid sequence of SEQ ID NO. 43.

In some embodiments, the nucleotide sequence of SEQ ID NO. 39 may be used to encode the recombinant FVIII protein or the variant thereof having an amino acid sequence of SEQ ID NO. 44.

In some embodiments, the nucleotide sequence of SEQ ID NO. 54 may be used to encode the recombinant FVIII protein or the variant thereof having an amino acid sequence of SEQ ID NO.53.

In some embodiments, the nucleotide sequence of SEQ ID NO. 56 may be used to encode the recombinant FVIII protein or the variant thereof having an amino acid sequence of SEQ ID NO. 50.

In some embodiments, the nucleotide sequence of SEQ ID NO. 58 may be used to encode the recombinant FVIII protein or the variant thereof having an amino acid sequence of SEQ ID NO. 52.

In some embodiments, the nucleotide sequence of SEQ ID NO. 60 may be used to encode the recombinant FVIII protein or the variant thereof having an amino acid sequence of SEQ ID NO. 51.

In a sixth aspect, a recombinant gene vector is provided.

A recombinant gene vector, comprising a nucleotide sequence encoding the recombinant FVIII protein or the variant thereof of the fourth aspect, or the nucleotide sequence of the fifth aspect.

In some embodiments, the recombinant gene vector may include a non-viral vector or a viral vector.

In some embodiments, the non-viral vector may be selected from a standard plasmid or other circular expression cassettes.

In some embodiments, the viral vector may be selected from a retroviral vector, a lentiviral vector, an adenoviral vector and an adeno-associated viral vector.

In some preferred embodiments, the non-viral vector may be selected from a minicircle DNA vector.

In a seventh aspect, a parental plasmid for use in the production of a minicircle DNA is provided.

A parental plasmid for use in the production of a minicircle DNA, comprising a plasmid vector containing the nucleotide sequence of the fifth aspect.

In some embodiments, the parental plasmid for use in the production of a minicircle DNA is formed by inserting in a plasmid vector a promoter nucleotide sequence, an enhancer nucleotide sequence, a multiple cloning site nucleotide sequence, a polyA signal nucleotide sequence, and a DNA fragment of a gene of interest, wherein the DNA fragment of the gene of interest is located between the restriction endonuclease cleavage sites of the multiple cloning site, and the DNA fragment of the gene of interest comprises the nucleotide sequence of the fifth aspect.

In some embodiments, the promoter may be a CMV promoter, and the nucleotide sequence thereof may be SEQ ID NO. 29.

In some embodiments, the enhancer nucleotide sequence may be SEQ ID NO. 30.

In some embodiments, the multiple cloning site nucleotide sequence may be SEQ ID NO. 31.

In some embodiments, the polyA signal may be selected from a bovine growth hormone polyA signal, a human growth hormone polyA signal, or an SV40 polyA signal.

In some embodiments, the bovine growth hormone polyA signal nucleotide sequence may be SEQ ID NO. 32.

In some embodiments, the DNA fragment of the gene of interest may also comprise a Kozak sequence and a sequence encoding a human FVIII gene signal peptide. The Kozak sequence is advantageous for enhancing transcription of the gene of interest.

In some embodiments, the nucleotide sequence of the Kozak sequence may be SEQ ID NO. 63 (GCCACC).

In some embodiments, the sequence encoding a human FVIII gene signal peptide may be SEQ ID NO. 33 or SEQ ID NO. 34.

In some embodiments, the plasmid vector is selected from a pMC.BESPX plasmid or a p2ΦC31 plasmid.

In some embodiments, the promoter nucleotide sequence, enhancer nucleotide sequence, multiple cloning site nucleotide sequence, and polyA signal nucleotide sequence are inserted into the plasmid at the recombination sites attB and attP of the plasmid.

In some embodiments, the DNA fragment of the gene of interest is inserted into the plasmid at the restriction endonuclease cleavage sites AgeI and EcoRV of the multiple cloning site.

In an eighth aspect, a method for preparing a minicircle DNA is provided.

A method for preparing a minicircle DNA, comprising: transforming the parental plasmid of the seventh aspect into a host cell, performing induction to allow the parental plasmid to produce a minicircle DNA and a backbone DNA by site-specific recombination at specific recombination sites, and extracting the minicircle DNA using a plasmid DNA purification kit.

In some embodiments, the backbone DNA is linearized within the host cell and subsequently degraded.

In some embodiments, the induction may include induction with L-arabinose.

In a ninth aspect, a minicircle DNA is provided.

A minicircle DNA obtained according to the preparation method of the eighth aspect. The minicircle DNA provided by the ninth aspect of the present disclosure has the advantages such as high expression level, good effect in secondary administration, simple preparation, and good therapeutic effect, and solves the problems of difficulty in production (difficulty in packaging), limitation in application range (failure to be used in children, failure to perform secondary administration), and safety risk (carcinogenesis due to random integration) of AAV.hf8, that is, the advantages such as simple production, wide application range (capability of being used in children, good efficacy in secondary administration), good safety, and long-term expression in vivo are provided.

In some embodiments, the nucleotide sequence of the minicircle DNA may be selected from SEQ ID NO. 25, SEQ ID NO. 26, SEQ ID NO. 27, SEQ ID NO. 28, SEQ ID NO. 46, SEQ ID NO. 47, SEQ ID NO. 49, SEQ ID NO. 55, SEQ ID NO. 57, SEQ ID NO. 59 or SEQ ID NO. 61.

In a tenth aspect, a host cell is provided.

A host cell, comprising a nucleotide sequence encoding the recombinant FVIII protein or the variant thereof of the fourth aspect, the nucleotide sequence of the fifth aspect, the recombinant gene vector of the sixth aspect or the minicircle DNA of the ninth aspect.

In some embodiments, the host cell may include a bacterial cell, a yeast cell, an insect cell or a mammalian cell. In some embodiments, the host cell is selected from *Escherichia coli.* In some embodiments, the host cell is selected from *Escherichia coli* ZYCY10P3S2T.

In an eleventh aspect, a pharmaceutical composition is provided.

A pharmaceutical composition, comprising the recombinant FVIII protein or the variant thereof of the fourth aspect, the recombinant FVIII protein or the variant thereof encoded by the nucleotide sequence of the fifth aspect, the recombinant gene vector of the sixth aspect or the minicircle DNA obtained according to the preparation method of the eighth aspect or the minicircle DNA of the ninth aspect, and a pharmaceutically acceptable auxiliary material or carrier. The pharmaceutical composition provided by the eleventh aspect of the present disclosure has the advantages such as high expression level, good effect in secondary administration, simple preparation, and good therapeutic effect, and solves the problems of difficulty in production (difficulty in packaging), limitation in application range (failure to be used in children, failure to perform secondary administration), and safety risk (carcinogenesis due to random integration) of AAV.hf8, that is, the advantages such as simple production, wide application range (capability of being used in children, good efficacy in secondary administration), good safety, and long-term expression in human bodies are provided.

In a twelfth aspect, use is provided.

Use of the recombinant FVIII protein or the variant thereof of the fourth aspect, the nucleotide sequence of the fifth aspect, the recombinant gene vector of the sixth aspect, the parental plasmid of the seventh aspect, the minicircle DNA obtained according to the preparation method of the eighth aspect, the minicircle DNA of the ninth aspect, the host cell of the tenth aspect or the pharmaceutical composition of the eleventh aspect for the preparation of a drug for treating a disease.

In some embodiments, the disease may be selected from a hereditary genetic defect disease.

In some embodiments, the disease may be a disease caused by coagulation factor deficiency.

In some embodiments, the disease may be a disease caused by a deficiency in FVIII.

In some embodiments, the disease may be hemophilia.

In some embodiments, the hemophilia is moderate hemophilia or severe hemophilia.

### Beneficial Effects

Compared with the prior art, a certain embodiment of the present disclosure has at least one of the following beneficial technical effects:
(1) The recombinant gene vector, minicircle DNA or pharmaceutical composition provided by the present disclosure, or the recombinant FVIII protein or the variant thereof using the linker peptide provided by the present disclosure has the advantages such as high expression level, good effect in secondary administration, simple preparation, good therapeutic effect, and long-term expression in vivo.
(2) The problems of difficulty in production (difficulty in packaging), limitation in application range (failure to be used in children, failure to perform secondary administration), and safety risk (carcinogenesis due to random integration) of AAV.hf8 are solved, that is, the advantages such as simple production, wide application range (capability of being used in children, good efficacy in secondary administration), good safety, and long-term expression in vivo are provided.

### Definition of Terms:

The term "room temperature" means ambient temperature, which may be 20°C-30°C; in some embodiments, the temperature is 22°C-28°C; in some embodiments, the temperature is 24°C-26°C; in some embodiments, the temperature is 25°C.

In the description of the description of the present disclosure, the description of reference to terms such as "an embodiment", "some embodiments", "examples", "specific examples", or "some examples" means that specific features, structures, materials or characteristics described in combination with the embodiment(s) or example(s) are comprised in at least one embodiment or example of the present disclosure. In the present description, the schematic expressions of the above terms do not necessarily refer to the same embodiment or example. Moreover, the specific features, structures, materials or characteristics described can be combined in any one or more embodiments or examples in any suitable manner. In addition, without any contradiction, a person skilled in the art may incorporate and combine different embodiments or examples and features of the different embodiments or examples described in the present description.

Generally, the fusion protein of the present disclosure is prepared by a method of biosynthesis. The nucleotide sequence according to the present disclosure, a person skilled in the art can conveniently use various known methods to prepare the encoding nucleic acid of the present disclosure. These methods are for example but not limited to: PCR, artificial synthesis of DNA, etc. Specific methods can refer to J. Sambrook, "Molecular Cloning: A Laboratory Manual". As an embodiment of the present disclosure, a method comprising segmented synthesis of nucleotide sequences, followed by overlap extension PCR can be used for constructing the encoding nucleic acid sequence of the present disclosure.

The term "parental plasmid" refers to the original plasmid that contains the expression cassette of a gene of interest, and a backbone DNA, which parental plasmid can produce a minicircle DNA. The plasmid produces the minicircle DNA after site-specific DNA recombination.

The term "fusion protein" generally refers to a protein obtained by the fusion of two or more proteins or polypeptides. Gene or nucleic acid molecules encoding two or more proteins or polypeptides may be linked to each other to form a fusion gene or fusion nucleic acid molecule. The fusion gene or fusion nucleic acid molecule may encode the fusion protein. Translation of the fusion gene results in a single polypeptide having the properties of at least one, or even each, of the two or more proteins or polypeptides prior to fusion. Recombinant fusion proteins are artificially created by recombinant DNA techniques for biological research or therapy. Recombinant fusion proteins are proteins created by genetic engineering of fusion genes. The present disclosure relates to recombinant fusion proteins, and the terms "fusion proteins" and "recombinant fusion proteins" are used herein with the same meaning. The fusion protein described herein generally comprises at least two domains (A and C), and optionally a third component, a linker between the two domains. The generation of recombinant fusion proteins is known in the art, and usually involves removal of a stop codon from a cDNA sequence encoding a first protein or polypeptide, followed by attachment of a cDNA sequence encoding a second protein in an in-frame manner by ligation or overlap extension PCR. The DNA sequence will then be expressed by the cell as a single protein. The protein can be engineered to include the complete sequence of two original proteins or polypeptides, or only a portion of either.

In the present application, the "pharmaceutical composition" may conveniently be presented in unit dosage form and may be prepared by any of the methods well known in the pharmaceutical field. All methods include the step of bringing into association the active agent with the carrier which constitutes one or more accessory ingredients. In general, the compositions are prepared by uniformly and intimately bringing into association the active compound with a liquid carrier, a finely divided solid carrier, or both.

In the present application, the term "recombinant gene vector" generally refers to a nucleic acid molecule capable of self-replication in a suitable host, which transfers the inserted nucleic acid molecule into and/or between host cells. The recombinant gene vector may include a vector primarily used for inserting DNA or RNA into a cell, a recombinant gene vector primarily used for replicating DNA or RNA, and a recombinant gene vector primarily used for the transcriptional and/or translational expression of DNA or RNA. The recombinant gene vector also include a recombinant gene vector having a variety of the above-mentioned functions. The recombinant gene vector may be a polynucleotide that can be transcribed and translated into a polypeptide when introduced into a suitable host cell. Generally, the recombinant gene vector may produce a desired expression product by culturing a suitable host cell comprising the recombinant gene vector.

In the present application, the term "mutant" may refer to any naturally occurring or engineered molecule that comprises one or more nucleotide or amino acid mutations.

In the present application, the term "nucleotide" refers to a ribonucleotide, deoxynucleotide or a modified form of either type of nucleotide, and combinations thereof.

The term "host cell" used herein refers to a prokaryotic or eukaryotic cell into which a recombinant expression vector can be introduced. The term "transformed" or "transfected" used herein refers to the introduction of a nucleic acid (e.g., a vector) into a cell by various techniques known in the art. Suitable host cells can be transformed or transfected with the DNA sequence of the present disclosure, and can be used for the expression and/or secretion of the target protein.

The term "identity", when used herein to describe an amino acid sequence or nucleic acid sequence relative to a reference sequence, can be determined using the formula described by Karlin and Altschul (Proc. Natl. Acad. Sci. USA 87: 2264-2268, 1990, modified as in Proc. Natl. Acad. Sci. USA 90:5873-5877, 1993). The formula is incorporated into the Basic Local Alignment Search Tool (BLAST) program of Altschul et al. (J. Mol. Biol. 215: 403-410, 1990). The percent identity between sequences can be determined using the latest version of BLAST as of the filing date of the present application.

The term "at least 80%-99% sequence identity" refers to at least 80%-99%, at least 81%-99%, at least 82%-99%, at least 83%-99%, at least 84%-99%, at least 85%-99%, at least 86%-99%, at least 87%-99%, at least 88%-99%, at least 89%-99%, at least 90%-99%, at least 91%-99%, at least 92%-99%, at least 93%-99%, at least 94%-99%, at least 95%-99%, at least 96%-99%, at least 97%-99%, at least 98%-99%, or at least 99% sequence identity to the respective reference sequence.

In the present disclosure, the "MC.BDD-FVIII-L1" or "MC.BDD-FVIII-L1 minicircle DNA" represents the same meaning, and the meanings of the other terms beginning with "MC.BDD", and so on, for example, the "MC.BDD-FVIII-L2" or "MC.BDD-FVIII-L2 minicircle DNA" represents the same meaning.

### DETAILED DESCRIPTION

In order to enable those skilled in the art to understand the technical solutions of the present disclosure better, some non-limiting examples are further disclosed below to further illustrate the present disclosure in detail.

All the reagents used in the present disclosure can be commercially available or prepared by the method described in the present disclosure.

The FVIII concentration in normal human plasma ranges from 100 to 200 ng/mL, and 200 ng/mL is defined as 100% of the normal level (Reference: J Biol Chem 2001, 276:46340-46346).

In the examples, the amino acid sequences and nucleotide sequences of various connecting peptides, FVIII proteins and minicircles are as follows:

**Table 1: Amino acid sequences and nucleotide sequences of various connecting peptides, FVIII proteins, minicircles, etc.**

| | |
|---|---|
| The amino acid sequence of L1 linker: SEQ ID NO.1 | The amino acid sequence of L2 linker: SEQ ID NO.2 |
| The nucleotide sequence of L1 linker: SEQ ID NO.3 | The nucleotide sequence of L2 linker: SEQ ID NO.4 |
| The amino acid sequence of BDD-FVIII-L1: SEQ ID NO.5 | The amino acid sequence of BDD-FVIII-L2: SEQ ID NO.6 |
| The nucleotide sequence- BDD-FVIII-L1: SEQ ID NO.7 | The nucleotide sequence- BDD-FVIII-L2: SEQ ID NO.8 |
| The amino acid sequence of v3 linker: SEQ ID NO.9 | The amino acid sequence of N8 linker: SEQ ID NO.10 |
| The amino acid sequence of BDD-FVIII-SQ-CO2: SEQ ID NO.11 | The amino acid sequence of BDD-FVIII-v3: SEQ ID NO.12 |
| The amino acid sequence of BDD-FVIII-N8: SEQ ID NO.13 | The amino acid sequence of BDD-FVIII-L3: SEQ ID NO.14 |
| The nucleotide sequence of BDD-FVIII-SQ-CO2: SEQ ID NO.15 | The nucleotide sequence of BDD-FVIII-v3: SEQ ID NO.16 |
| The nucleotide sequence: BDD-FVIII-N8: SEQ ID NO.17 | The nucleotide sequence: BDD-FVIII-L3: SEQ ID NO.18 |
| The nucleotide sequence of BDD-FVIII-CTP-Fc: SEQ ID NO.19 | The amino acid sequence of BDD-FVIII-CTP-Fc: SEQ ID NO.20 |
| The amino acid sequence of BDD-FVIII-L1-X5: SEQ ID NO.21 | The nucleotide sequence of BDD-FVIII-L1-X5: SEQ ID NO.22 |
| The amino acid sequence of BDD-FVIII-L2-X5: SEQ ID NO.23 | The nucleotide sequence of BDD-FVIII-L2-X5: SEQ ID NO.24 |
| The nucleotide sequence of MC.BDD-FVIII-L1: SEQ ID NO.25 | The nucleotide sequence of MC.BDD-FVIII-L2: SEQ ID NO.26 |
| The nucleotide sequence of MC.BDD-FVIII-L1-X5: SEQ ID NO.27 | The nucleotide sequence of MC.BDD-FVIII-L2-X5: SEQ ID NO.28 |
| The nucleotide sequence of CMV promoter: SEQ ID NO.29 | The nucleotide sequence of CMV enhancer: SEQ ID NO.30 |
| The nucleotide sequence of multiple cloning site: SEQ ID NO.31 | The nucleotide sequence of bovine growth hormone polyA signal: SEQ ID NO.32 |
| The nucleotide sequence of human FVIII gene signal peptide: SEQ ID NO.33 | The nucleotide sequence of human FVIII signal peptide (unoptimized): SEQ ID NO.34 |
| The nucleotide sequence of BDD-FVIII-SQ-WT: SEQ ID NO.35 | The nucleotide sequence of BDD-FVIII-v3-WT: SEQ ID NO.36 |
| The nucleotide sequence of BDD-FVIII-N8-WT: SEQ ID NO.37 | The nucleotide sequence of BDD-FVIII-L1-WT: SEQ ID NO.38 |
| The nucleotide sequence of BDD-FVIII-L2-WT: SEQ ID NO.39 | The amino acid sequence of BDD-FVIII-SQ-WT: SEQ ID NO.40 |
| The amino acid sequence of BDD-FVIII-v3-WT: SEQ ID NO.41 | The amino acid sequence of BDD-FVIII-N8-WT: SEQ ID NO.42 |
| The amino acid sequence of BDD-FVIII-L1-WT: SEQ ID NO.43 | The amino acid sequence of BDD-FVIII-L2-WT: SEQ ID NO.44 |
| The nucleotide sequence of BDD-FVIII-SQ-CO1: SEQ ID NO.45 | MC.BDD-FVIII-L1-WT The nucleotide sequence of: SEQ ID NO.46 |
| The nucleotide sequence of MC.BDD-FVIII-L2-WT: SEQ ID NO.47 | The nucleotide sequence of MC.BDD-FVIII-SQ-CO1: SEQ ID NO.48 |
| The nucleotide sequence of MC.BDD-FVIII-SQ-CO2: SEQ ID NO.49 | The amino acid sequence of BDD-FVIII-L1_{R1645H}: SEQ ID NO.50 |
| The amino acid sequence of BDD-FVIII_{F309S}-L1: SEQ ID NO.51 | The amino acid sequence of BDD-FVIII_{F309S}-L1_{R1645H}: SEQ ID NO.52 |
| The amino acid sequence of BDD-FVIII_{F309S}-L2: SEQ ID NO.53 | The nucleotide sequence of BDD-FVIII_{F309S}-L2: SEQ ID NO.54 |
| The nucleotide sequence of MC.BDD-FVIII_{F309S}-L2: SEQ ID NO.55 | The nucleotide sequence of BDD-FVIII-L1_{R1645H}: SEQ ID NO.56 |
| The nucleotide sequence of MC.BDD-FVIII-L1_{R1645H}: SEQ ID NO.57 | The nucleotide sequence of BDD-FVIII_{F309S}-L1_{R1645H}: SEQ ID NO.58 |
| The nucleotide sequence of MC.BDD-FVIII_{F309S}-L1_{R1645H}: SEQ ID NO.59 | The nucleotide sequence of BDD-FVIII_{F309S}-L1: SEQ ID NO.60 |
| The nucleotide sequence of MC.BDD-FVIII_{F309S}-L1: SEQ ID NO.61 | The amino acid sequence of SQ linker: SEQ ID NO.62 |
| The nucleotide sequence of Kozak sequence: SEQ ID NO.63 | / |

The sequence with "WT" in its sequence name means that its BDD-FVIII coding sequence is a coding sequence obtained by almost completely deletion of the B region (reserving the SQ linker) from the wild-type human FVIII coding sequence (serial number: NM_000132), and the sequence with "CO1" or "CO2" means that its BDD-FVIII coding sequence is a coding sequence obtained by codon optimization of the wild-type human FVIII coding sequence (serial number: NM_000132) from which the region B is almost completely deleted (reserving the SQ linker).
SEQ ID NO.63: GCCACC

### Example 1: Construction of minicircle DNA parental plasmids (PPs)

The minicircle DNA parent plasmids were constructed by the following steps:
(1) The nucleotide sequences of CMV promoter (SEQ ID NO.29), CMV enhancer (SEQ ID NO.30), multiple cloning site (MCS) (SEQ ID NO.31), and bovine growth hormone polyadenylation signal (bpA) (SEQ ID NO.32) were inserted between the attB and attP recombination sites of the minicircle DNA cloning vector (pMC.BESPX) to construct the vector pMC.CMV-MCS-bpA.
(2) The DNA fragments of the target genes were synthesized, including the Kozak sequence (SEQ ID NO.63) that can enhance the transcription of the target gene, the human FVIII gene signal peptide coding sequence (SEQ ID NO.33), and the BDD-FVIII coding sequence containing the SQ linker (unoptimized) (SEQ ID NO.35). The synthesized DNA fragments were then cloned between the AgeI/EcoRV restriction sites of the pMC.CMV-MCS-bpA multiple cloning site to construct the minicircle DNA parent plasmid of the wild-type BDD-FVIII containing the SQ linker (pMC.BDD -FVIII-SQ-WT).
(3) Referring to the operations (1) and (2), the SQ linker-containing BDD-FVIII coding sequence (unoptimized) (SEQ ID NO.35) was replaced with the optimized SQ linker-containing BDD-FVIII coding sequence-CO1 (SEQ ID NO.45), the optimized SQ linker-containing BDD-FVIII coding sequence-CO2 (SEQ ID NO.15), the optimized v3 linker-containing BDD-FVIII coding sequence (SEQ ID NO.16), the optimized N8 linker-containing BDD-FVIII coding sequence (SEQ ID NO.17), the optimized L1 linker-containing BDD-FVIII coding sequence (SEQ ID NO.7), the optimized L2 linker-containing BDD-FVIII coding sequence (SEQ ID NO.8), the optimized L3 linker-containing BDD-FVIII coding sequence (SEQ ID NO.18), the v3 linker-containing BDD-FVIII coding sequence (SEQ ID NO.36), the N8 linker-containing BDD-FVIII coding sequence (SEQ ID NO.37), the L1 linker-containing BDD-FVIII coding sequence (SEQ ID NO.38), and the L2 linker-containing BDD-FVIII coding sequence (SEQ ID NO.39), to respectively construct different minicircle DNA parent plasmids such as pMC.BDD-FVIII-SQ-CO1, pMC.BDD-FVIII-SQ-CO2, pMC.BDD-FVIII-v3, pMC.BDD-FVIII-N8, pMC.BDD-FVIII-L1, pMC.BDD-FVIII-L2, pMC.BDD-FVIII-L3, pMC.BDD-FVIII-v3-WT, pMC.BDD-FVIII-N8-WT, pMC.BDD-FVIII-L1-WT, pMC.BDD-FVIII-L2-WT (the nucleotide sequences of various minicircle DNA parent plasmids refer are shown in Table 1).

### Example 2: Production of Minicircle DNA

The minicircle DNA was produced by the following steps:
(1) The parent plasmids (pMC.BDD-FVIII-SQ-WT, pMC.BDD-FVIII-SQ-CO1, pMC.BDD-FVIII-SQ-CO2, pMC.BDD-FVIII-v3, pMC.BDD-FVIII-N8, pMC.BDD-FVIII-L1, pMC.BDD-FVIII-L2, pMC.BDD-FVIII-L3, pMC.BDD-FVIII-L1-WT, and pMC.BDD-FVIII-L2-WT) were respectively transformed into the genetically engineered *E. coli* ZYCY10P3S2T.
(2) The positive monoclonal colonies were picked, inoculated into an LB medium or TB medium, and cultured on a shaker at 37°C for 12 h to 16 h.
(3) L-arabinose was added (to a final L-arabinose concentration of 0.2 wt%) to perform an induction at an induction temperature of 30°C to 32°C for an induction time of 6-8 hours so that parent plasmids were induced to undergo site-specific DNA recombination to form minicircle DNA and skeleton DNA, wherein the skeleton DNA was linearized in the bacterial body and then degraded.
(4) The respective minicircle DNA (wherein the nucleotide sequences of various minicircle DNAs are shown in Table 1) was extracted using a plasmid DNA purification kit (QIAGEN EndoFree Plasmid Mega Kit, Qiagen, Germany): MC.BDD-FVIII-SQ-WT, MC.BDD-FVIII-SQ-CO1, MC.BDD-FVIII-SQ-CO2, MC.BDD-FVIII-v3, MC.BDD-FVIII-N8, MC.BDD-FVIII-L1, MC.BDD-FVIII-L2, MC.BDD-FVIII-L3, MC.BDD-FVIII-L1-WT, and MC.BDD-FVIII-L2-WT.

### Example 3: Cell Transfection

(1) 293T cells were inoculated into a 6-well plate at a density of 1x10⁶cells per well;
(2) The cells were cultured in a DMEM medium containing 10% fetal bovine serum at 37°C, 5% CO₂ for 24 hours to obtain cells to be transfected;
(3) The serum-containing DMEM medium was discarded and replaced with a serum-free expression medium (Expi293, Thermo Fisher Scientific), and cultured in an incubator at 37°C for additional 4 hours;
(4) The minicircle DNA was transfected into the cells to be transfected at an amount of 1.2 µg/well using a transfection reagent (X-tremeGENE HP DNA Transfection Reagent, Roche) to obtain the recombinant cells; and
(5) The recombinant cells were cultured for 72 hours, and the cell culture supernatant was collected for subsequent testing.

### Example 4: Western blot detection

(1) Preparation of samples: A reducing loading buffer was respectively added into the collected cell culture supernatant, a negative control sample (transfection reagent), and a positive control sample (hFVIII plasma extract; Guizhou Taibang Biological Products Co., Ltd.);
(2) Electrophoresis: The sample prepared in step (1) was supplemented with an appropriate amount of loading buffer, and then heated in boiling water for 3-5 minutes to denature the protein, cooled, loaded into to the SDS-PAGE gel loading hole, and electrophoresed at 80-100V for 1 hour;
(3) Transfer: The sample was transferred using a wet transfer-blot (Bio-Rad) at 300 mA for 1 hour to transfer the protein from the SDS-PAGE gel to the PVDF membrane;
(4) Blocking: The PVDF membrane was rinsed, and blocked by adding a blocking solution;
(5) Antibody incubation: A diluted HRP-labeled anti-human FVIII antibody (Affinity Biologicals) was added and incubated at room temperature for 1 hour; and
(6) Development: The protein was detected using an ECL chemiluminescence reagent (Cell Signaling).

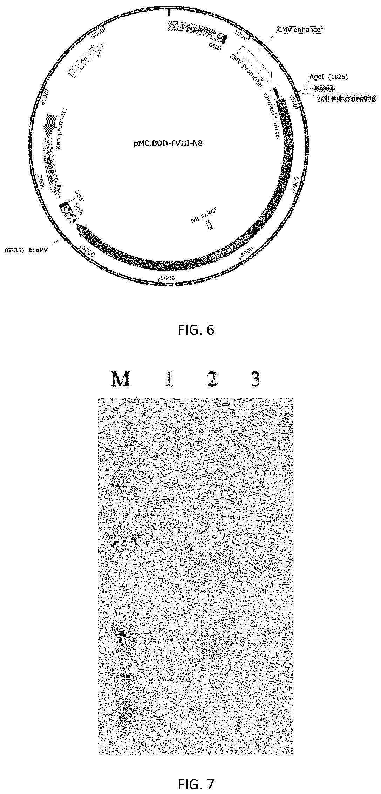

Results: The detection results of MC.BDD-FVIII-SQ-CO2 are shown in FIG. 7.

### Example 5: Investigation of BDD-FVIII codon-optimized sequence

The wild-type human FVIII coding sequence (sequence number: NM_000132) was obtained from the Genbank database, and the B region was almost completely deleted (reserving the SQ linker) to construct the wild-type BDD-FVIII-SQ-WT (SEQ ID NO.35). The wild-type BDD-FVIII was subjected to codon optimization using two optimization algorithms to give two optimized BDD-FVIII sequences BDD-FVIII-SQ-CO1 (SEQ ID NO.45) and BDD-FVIII-SQ-CO2 (SEQ ID NO.15), respectively. In accordance with the method as described in Example 1, MC.BDD-FVIII-SQ-WT, MC.BDD-FVIII-SQ-CO1, and MC.BDD-FVIII-SQ-CO2 were prepared (the nucleotide sequences of various minicircles are shown in Table 1). 293 cells were transfected with MC.BDD-FVIII-SQ-WT, MC.BDD-FVIII-SQ-CO1, and MC.BDD-FVIII-SQ-CO2, respectively. After 72 hours, the cell culture supernatant was collected and the expression level of the target protein was detected by ELISA.

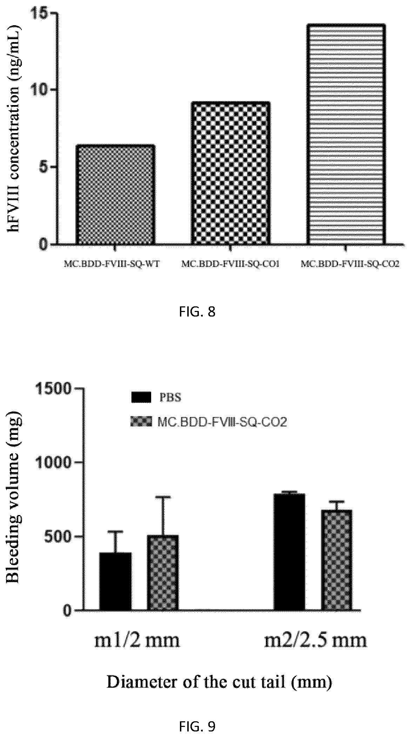

Results: As shown in FIG. 8.

Conclusions: The expression levels of the two optimized vectors are significantly improved, and the second optimized version (BDD-FVIII-CO2) has a greater improvement, that is, more than 1 times.

### Comparative Example 1: Investigation of In vivo Expression and Activity of MC.BDD-FVIII-SQ-CO2

### Plasma expression level of MC.BDD-FVIII-SQ-CO2 in wild-type C57 mice:

Operations: MC.BDD-FVIII-SQ-CO2 was intramuscularly injected into wild-type C57 mice at a dose of 30 µg/mouse, and the plasma expression level was measured by ELISA.

Results: The plasma expression levels were measured by ELISA, and it is found that there was only weak expression (1-2 ng/mL).

### Therapeutic effect of MC.BDD-FVIII-SQ-CO2 on mice with hemophilia A with F8 gene knockout (F8-KO mice):

Operations: Six 12-week-old male F8-KO mice were randomly divided into two groups, with 3 in each group. Each mouse in the treatment group was intramuscularly injected with 30 µg/50 µL of the minicircle DNA (MC.BDD-FVIII-SQ-CO2) solution (30 µg minicircle DNA in 50 µl solution), and each mouse in the blank control group was intramuscularly injected with 50 µL PBS. After the intramuscular injection, a pulsed electric field was applied (TERESA in vivo gene introduction instrument; parameters: 36 V, 10 ms, 1 Hz). 1 month (m1) and 2 months (m2) after the injection, the mice were subjected to tail cutting experiments in twice at sites where the mouse tail had diameter of 2 mm and 2.5 mm, respectively, and the bleeding volume was recorded. After the tail cutting, the wound was cauterized with AgNO₃ to stop the bleeding.

Results: As shown in FIG. 9, after a period of time, the mice in the minicircle DNA (MC.BDD-FVIII-SQ-CO2) injection group and the mice in the untreated control group (no minicircle DNA, only injected with equal volume of PBS buffer) were subjected to tail cutting (at the sites where the tail had a diameter of 2 mm if cut at 1 month; and a diameter of 2.5 mm if cut at 2 months) to compare the bleeding volume s therebetween. It is found that the two groups had comparable bleeding volume without statistical difference, indicating that MC.BDD-FVIII-SQ-CO2 treatment failed to improve the coagulation function of mice with hemophilia A.

### Example 6: Measurement of target protein expression level by ELISA

The cell culture supernatants of MC.BDD-FVIII-L1, MC.BDD-FVIII-L2, MC.BDD-FVIII-L3, MC.BDD-FVIII-v3, and MC.BDD-FVIII-N8 obtained in Example 3 were respectively measured for their target protein expression levels by ELISA.

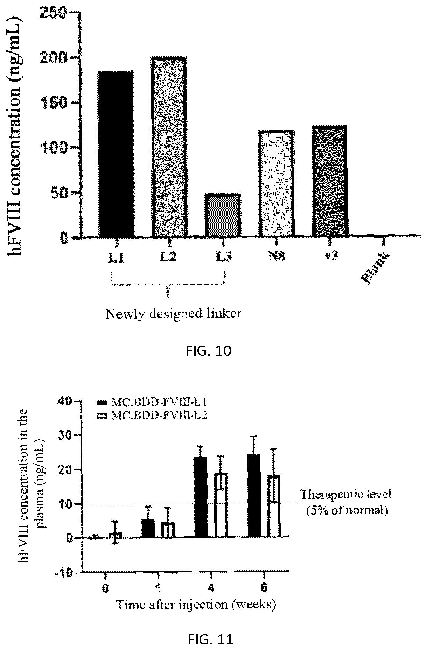

Results: As shown in FIG. 10, the target protein expression levels of MC.BDD-FVIII-L1 and MC.BDD-FVIII-L2 are significantly higher than those of other FVIII minicircle DNA expression vectors. The relative levels are as follows:
(1) The target protein expression level ratios of MC.BDD-FVIII-L1 to MC.BDD-FVIII-N8 and MC.BDD-FVIII-v3:
   MC.BDD-FVIII-L1: MC.BDD-FVIII-N8 = 1.867;
   MC.BDD-FVIII-L1: MC.BDD-FVIII-v3 = 1.849;
   MC.BDD-FVIII-L1 is approximately 87% and 85% higher than MC.BDD-FVIII-N8 and MC.BDD-FVIII-v3, respectively.
(2) The target protein expression level ratios of MC.BDD-FVIII-L2 to MC.BDD-FVIII-N8 and MC.BDD-FVIII-v3:
   MC.BDD-FVIII-L2: MC.BDD-FVIII-N8 = 1.924;
   MC.BDD-FVIII-L2: MC.BDD-FVIII-v3 = 1.906;
   MC.BDD-FVIII-L2 is approximately 92% and 91% higher than MC.BDD-FVIII-N8 and MC.BDD-FVIII-v3, respectively.
(3) The target protein expression level ratios of MC.BDD-FVIII-L3 to MC.BDD-FVIII-N8 and MC.BDD-FVIII-v3:
   MC.BDD-FVIII-L3: MC.BDD-FVIII-N8 = 0.443;
   MC.BDD-FVIII-L3: MC.BDD-FVIII-v3 = 0.439;
   MC.BDD-FVIII-L3 is appropriately equivalent to 44% of MC.BDD-FVIII-N8 or MC.BDD-FVIII-v3.
   MC.BDD-FVIII-v3: MC.BDD-FVIII-N8 = 1.01, and there is almost no difference between MC.BDD-FVIII-v3 and MC.BDD-FVIII-N8.
(4) The target protein expression level ratios of MC.BDD-FVIII-L1 to MC.BDD-FVIII-L2:
   MC.BDD-FVIII-L1: MC.BDD-FVIII-L2 = 0.97, and there is almost no difference between MC.BDD-FVIII-L1 and MC.BDD-FVIII-L2.

Conclusions: The FVIII minicircle DNA vector of linker peptide containing L1-linker or L2-linker has a higher expression level of the target protein than that of the FVIII minicircle DNA vector of another type of linker peptide.

### Example 7: Detection of In Vitro Coagulation Activity (One-Stage APTT Method)

Operations: The cell culture supernatants of MC.BDD-FVIII-L1 and MC.BDD-FVIII-L2 with the highest expression level of the target protein obtained in Example 3 were collected and sent to the Institute of Blood Transfusion, Chinese Academy of Medical Sciences for detection of the coagulation activity using the one-stage APTT method. The FVIII activity of normal pooled plasma (NPP) is defined as 100%.

Results: The measured activities of the expression products (the cell culture supernatant at 72h) of MC.BDD-FVIII-L1 and MC.BDD-FVIII-L2 were respectively 118.1% and 116% (the reference activity for a person with normal coagulation function ranges from 50% to 150%), indicating that the FVIII containing a linker peptide of L1-linker or L2-linker has good coagulation activity.

### Example 8: In Vivo Expression

### (1) Expression in normal mice

Operations: Ten 6-8-week-old Balb/c mice were randomly divided into two groups with 5 mice in each group so that there were 5 mice in the MC.BDD-FVIII-L1 group and 5 mice in the MC.BDD-FVIII-L2 group. Blood was collected one day before the injection of minicircle DNA, and plasma was separated and stored at -80°C, which was used as a blank control sample (W0). The mice in the two groups were intramuscularly injected with the MC.BDD-FVIII-L1 minicircle DNA and the MC.BDD-FVIII-L2 minicircle DNA at a dose of 30 µg/50 µL/mouse, and then a pulsed electric field was applied (TERESA in vivo gene introduction instrument; Parameters: 36 V, 10 ms, 1 Hz). After the injection, blood was collected periodically, and plasma was separated and stored at -80°C. The target protein expression level in the plasma sample was detected using a Human FVIII ELISA kit (VisuLize Factor VIII PLUS Antigen ELISA Kit, Affinity Biologicals Inc).

Results: As shown in FIG. 11. There is no expression in the blank control sample. 4 weeks after the injection, the MC.BDD-FVIII-L1 group and the MC.BDD-FVIII-L2 group have similar *in vivo* expression levels of MC.BDD-FVIII-L1 and MC.BDD-FVIII-L2 of up to 20 ng/mL or more (equivalent to 10% or more of the normal plasma FVIII concentration level in normal person (20 ng/mL)), which exceeds the significant therapeutic level that can significantly improve moderate or severe hemophilia to mild (>5%).

### (2) Expression in Beagle Dogs

Operations: 3 adult beagle dogs with weight of about 13 kg were subjected to blood collection before the injection of minicircle DNA, and plasma was separated and stored at -80°C, which was used as a blank control sample (W0). On the day of blood collection, each dog was continued to inject with 600 µg of MC.BDD-FVIII-L1 minicircle DNA, and then a pulsed electric field was applied (TERESA in vivo gene introduction instrument; parameters: 36 V, 10 ms, 1 Hz). Then, blood was collected every week, and plasma was separated and stored at -80°C. The target protein expression level in the plasma sample was detected using a Human FVIII ELISA kit (VisuLize Factor VIII PLUS Antigen ELISA Kit, Affinity Biologicals Inc), and then converted to a relative FVIII level in the Normal Pooled Plasma (NPP). The FVIII concentration in the Normal Pooled Plasma is about 200 ng/mL.

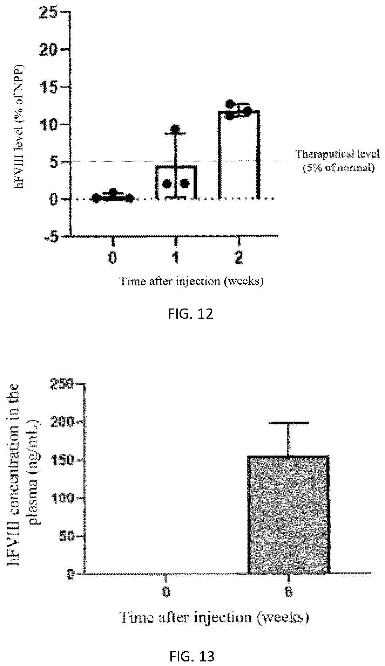

Results: As shown in FIG. 12. There is no expression in the blank control sample. One week after the injection of MC.BDD-FVIII-L1 minicircle DNA, the plasma hFVIII expression reached 5% (10 ng/mL) or more of the normal FVIII concentration level in the normal human plasma in one dog, but were relatively low in the other two dogs; and two weeks after the injection of MC.BDD-FVIII-L1 minicircle DNA, the expression levels gradually increased and all exceeded 10% (20 ng/mL) of the normal FVIII concentration level in normal human plasma.

### Example 9: Activity in Mice with Hemophilia A

### (1) In vivo expression level of MC.BDD-FVIII-L1 in mice with hemophilia A (F8-KO mice)

Operations: One day before the injection of minicircle DNA, five 6-8-week-old male F8-KO mice were subjected to blood collection and plasma was separated and stored at -80°C, which was used as a blank control sample (W0). All the mice were intramuscularly injected with the minicircle DNA (MC.BDD-FVIII-L1 minicircle DNA) at a dose of 30 µg/50 µL/mouse (30 µg minicircle DNA in 50 µL solution), and then a pulsed electric field was applied (TERESA in vivo gene introduction instrument; Parameters: 36 V, 10 ms, 1 Hz). After 6 weeks (W6), blood was collected, and plasma was separated and stored at -80°C. The target protein expression level in the plasma sample was detected using a Human FVIII ELISA kit (VisuLize Factor VIII PLUS Antigen ELISA Kit, Affinity Biologicals Inc).

Results: The results are shown in FIG. 13. There is no expression in the blank control sample. 6 weeks after the injection of MC.BDD-FVIII-L1 minicircle DNA, it is measured by ELISA that the plasma target protein level is up to 100 ng/mL or more, which has exceeded 50% (100ng/mL) of the normal FVIII concentration level in normal human plasma, indicating that it can functionally cure hemophilia and restore the normal coagulation function.

### (2) Treatment of MC.BDD-FVIII-L1 on Mice with Hemophilia A (F8-KO mice)

Operations: Ten 6-8-week-old male F8-KO mice were randomly divided into two groups including a blank control group (PBS) and a minicircle DNA treatment group (the MC treatment group, MC), with 5 mice in each group. Each mouse in the minicircle DNA treatment group was intramuscularly injected with MC.BDD-FVIII-L1 minicircle DNA solution at a dose of 30 µg/50 µL (30 µg minicircle DNA in 50 µL solution), and then a pulsed electric field was applied after the injection (TERESA in vivo Gene introduction instrument; parameters: 36 V, 10 ms, 1 Hz); and the mice in the blank control group were injected with an equal volume of PBS buffer by the same way. Additional five male C57 mice of the same age served as wild-type controls (WT) with normal coagulation function. 4 weeks after the injection, a tail-cutting experiment was performed at site where the tail had a diameter of 2 mm, and the bleeding volume within 15 minutes was recorded. After the experiment, the mice were sacrificed.

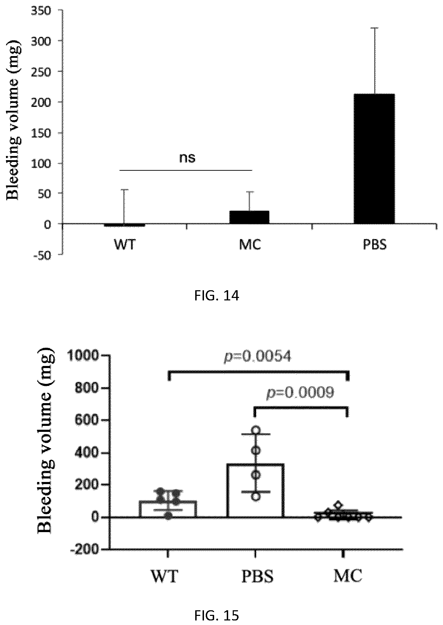

Results: As shown in FIG. 14, in comparison with the blank control group (PBS), the bleeding volume of the mice in the MC.BDD-FVIII-L1 minicircle DNA treatment group (the MC treatment group, MC) was significantly reduced, while the bleeding volume of the mice in the MC treatment group is comparable with the bleeding volume of the wild-type normal mice (WT) (no statistical difference, ns); indicating that the coagulation function of the mice with hemophilia A has returned to normal after the MC treatment.

### (3) Identification of Activity of MC.BDD-FVIII-L2 in Mice with Hemophilia A (F8-KO Mice)

Operations: Twenty 9-10-week-old male F8-KO mice were randomly divided into two groups: 5 mice in the blank control group (PBS) and 15 mice in the minicircle DNA injection group (the MC treatment group, MC). The mice in the MC treatment group were injected with MC.BDD-FVIII-L2 minicircle DNA solution (3 µg minicircle DNA dissolved in 1.8 mL PBS buffered solution) at a dose of 3 µg/ 1.8 mL/mouse (3 µg minicircle DNA in 1.8 mL solution) by ways of high-pressure injection through tail vein (1.8 mL of solution is rapidly injected through the tail vein within 5-8 seconds); and the mice in the blank control group were injected with an equal volume (1.8 mL) of PBS buffered solution by the same way. Additional five male C57 mice of the same age served as wild-type controls (WT) with normal coagulation function. 24 hours after the injection, a tail-cutting experiment was performed at a point where the diameter of the mouse tail was 2 mm, and the bleeding volume within 15 minutes was recorded; the mice were sacrificed after the experiment. One mouse in the blank control group died due to abnormal high-pressure injection operation and failed to undergo tail cutting.

Results: As shown in FIG. 15, in comparison with the blank control group (PBS), the bleeding volume of the mice in the MC.BDD-FVIII-L2 minicircle DNA injection group (the MC treatment group, MC) was significantly reduced, even less than the untreated wild-type normal mice (WT), indicating that MC.BDD-FVIII-L2 has extremely high coagulation activity in vivo.

### Example 10: Other modifications

### (1) Fc fusion

Fc fusion is important means to increase the half-life of FVIII. Molecules with longer half-lives of *in vivo* expression may result in more product accumulation in the body to achieve higher expression level. To verify this idea, we constructed the minicircle DNA expression vector (MC.BDD-FVIII-CTP-Fc) of the BDD-FVIIIFc fusion protein (SEQ ID NO.19) (the nucleotide sequence of the minicircle is shown in the Table 1).

Operations: Ten 6-8-week-old Balb/c mice were randomly divided into two groups: 5 mice in the minicircle DNA (MC.BDD-FVIII-CTP-Fc) injection group and 5 mice in the PBS blank control group (control). The mice in the minicircle group were intramuscularly injected with the minicircle DNA (MC.BDD-FVIII-CTP-Fc) at dose of 45 µg/50 µL/mouse (each mouse was injected with 50 µL comprising 45 µg minicircle DNA which is equimolar to 30 µg MC.BDD-FVIII), and each mouse in the blank control group was intramuscularly injected with 50 µL PBS. After the intramuscular injection, a pulsed electric field was applied (TERESA in vivo gene introduction instrument; Parameters: 36 V, 10 ms, 1 Hz). Then, blood was collected periodically, and plasma was separated and stored at -80°C. The target protein expression level in the plasma sample was detected using a Human FVIII ELISA kit (VisuLize Factor VIII PLUS Antigen ELISA Kit, Affinity Biologicals Inc).

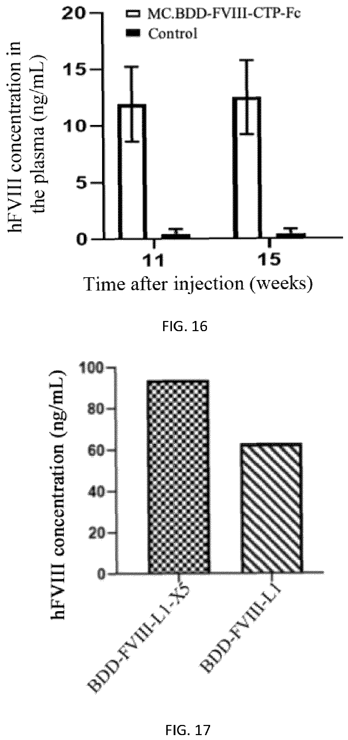

Results: As shown in FIG. 16. There is no expression in the blank control sample. 11-15 weeks after the injection of MC.BDD-FVIII-CTP-Fc minicircle DNA, it was found that the minicircle DNA of the BDD-FVIII-Fc fusion protein could not further increase the *in vivo* expression level (only about 10 ng/mL, which is partially less than that 5% (10 ng/mL) of the normal FVIII concentration level in normal human plasma).

### (2) X5 mutation

### Measurement of in vitro cell expression level of the target protein by ELISA:

Operations: Mutation X5 (i.e., five key amino acids in the A region of human FVIII are mutated to the amino acids at the corresponding sites of porcine FVIII; I86V/A108S/G132K/M147T/L152P) was introduced into the BDD-FVIII-L1 to give the BDD-FVIII-L1-X5 nucleotide sequence (SEQ ID NO.22) so as to construct the corresponding MC expression vector (the nucleotide sequences of various minicircles are shown in Table 1): MC.BDD-FVIII-L1-X5 minicircle DNA. MC.BDD-FVIII-L1 minicircle DNA and MC.BDD-FVIII-L1-X5 minicircle DNA were transfected into 293T cells, respectively. After 72 hours, the cell culture supernatant was collected, and the target protein level was measured by ELISA.

Results: As shown in FIG. 17, in comparison with the MC.BDD-FVIII-L1 minicircle DNA, the target protein level was improved after the introduction of the X5 mutation.

### Experiments in mice:

**Operations:** Ten 6-8-week-old Balb/c mice were randomly divided into two groups with 5 mice in each group. The mice in each group were intramuscularly injected with the minicircle DNA (MC.BDD-FVIII-L1 minicircle DNA or MC.BDD-FVIII-L1-X5 minicircle DNA) at a dose of 30 µg/50 µL/mouse (each mouse was injected with 50 µL containing 30 µg minicircle DNA). After the intramuscular injection, a pulsed electric field was applied (TERESA in vivo gene introduction instrument; parameters: 36 V, 10 ms, 1 Hz). 4 weeks after the injection, blood was collected and the plasma was separated. The target protein expression level in the plasma sample was detected using a Human FVIII ELISA kit (VisuLize Factor VIII PLUS Antigen ELISA Kit, Affinity Biologicals Inc).

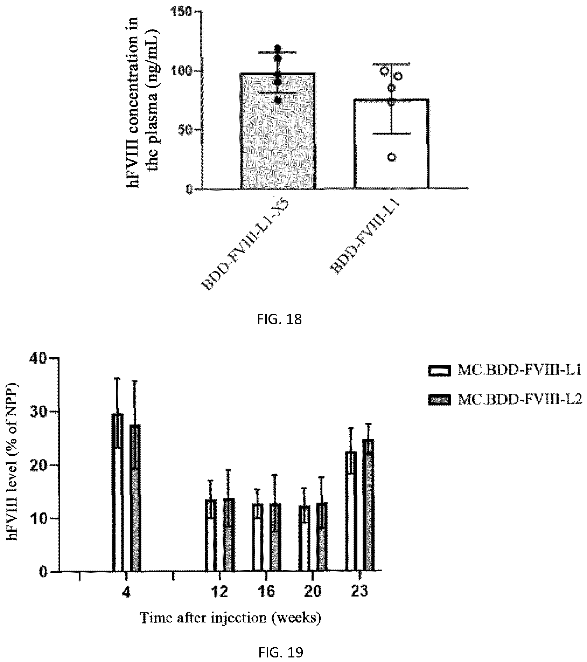

**Results:** As shown in FIG. 18, in comparison with MC.BDD-FVIII-L1 minicircle DNA, the target protein level was slightly improved after the introduction of the X5 mutation.

### (3) R1645H mutation

Siner et al. (Blood 2013, 121:4396-4403) reported that mutating the furin site at the C terminus of the SQ linker in the B region of BDD-FVIII was mutated (R₁₆₄₅-H₁₆₄₆-Q₁₆₄₇-R₁₆₄₈) results in that the BDD-FVIII is unable to be recognized and cleaved by the Furin protease so that the expression increases. Since the recognition pattern of the furin site is R-X-X-R (that is, the first and last amino acids are arginine, and the middle two amino acids are arbitrary), by mutating the arginine at position 1645 or 1648 to any other amino acid except arginine, it can theoretically achieve the purpose of failing to be recognized and cleaved by the Furin proteas. This purpose can also be achieved by deleting the furin site (including deleting all 4 amino acids of RHQR, or any 1, 2 or 3 amino acids therein).

In the present disclosure, the furin site of RHQR is retained at the C-terminal of the L1 linker. Accordingly, we mutate the arginine (Arg, R) at the 4^{th} position from the C-terminal of the L1 linker to histidine (His, H), that is, a R1645H mutation, to give a coding sequence BDD-FVIII-L1_{R1645H} of the BDD-FVIII-L1 (the nucleotide sequence is shown in Table 1), which was construct into a corresponding MC expression vector MC.BDD-FVIII-L1_{R1645H} (the nucleotide sequence of the minicircle is shown in Table 1).

### (4) F309S mutation

Swaroop et al (J Biol Chem 1997, 272:24121-24124) reported that the F309S mutation (the phenylalanine at position 309 of FVIII is mutated to serine) can promote FVIII secretion. Accordingly, we mutated the phenylalanine at position 309 of BDD-FVIII-L1, BDD-FVIII-L1_{R1645H}and BDD-FVIII-L2 to serine ( Ser, S) to respectively give the coding sequences of BDD-FVIII_{F309S}-L1, BDD-FVIII_{F309S}-L1_{R1645H}and BDD-FVIII_{F309S}-L2 (various nucleotide sequences ae shown in Table 1), which were constructed into the corresponding MC expression vectors MC.BDD-FVIII_{F309S}-L1, MC.BDD -FVIII_{F309S}-L1_{R1645H}and MC.BDD-FVIII_{F309S}-L2 (the nucleotide sequences of various minicircles are shown in Table 1).

### Example 11: Therapeutic effect of secondary administration

Operations: Ten 6-8-week-old Balb/c mice were randomly divided into two groups: 5 in the MC.BDD-FVIII-F1 group and 5 in the MC.BDD-FVIII-L2 group. Each mouse in the two groups was intramuscularly injected with the MC.BDD-FVIII-L1 minicircle DNA and the MC.BDD-FVIII-L2 minicircle DNA at a dose of 30 µg/50 µL/mouse (each mouse was injected with 50 µL containing 30 µg minicircle DNA), and then a pulsed electric field was applied (TERESA in vivo gene introduction instrument; parameters: 36 V, 10 ms, 1 Hz). On the 22^{nd} week (W22) after the injection, the mice were dosed by the same way. After the first and second injections, blood was collected periodically, and plasma was separated and stored at -80°C. The target protein expression level in the plasma sample was detected using a Human FVIII ELISA kit (VisuLize Factor VIII PLUS Antigen ELISA Kit, Affinity Biologicals Inc). And it was converted into the relative level of FVIII in normal pooled plasma (NPP). The FVIII concentration in the Normal Pooled Plasma is about 200 ng/mL.

Results: As shown in FIG. 19. After the injection of minicircle DNA, the expression levels in mice of both groups were above 20% (40 ng/mL) of the normal FVIII concentration level in normal human plasma on the 4^{th} week (W4). The expression levels were somewhat decreased on the 12^{th} week (W12), but could still be maintained at above 10% (20 ng/mL) of the normal level of normal human plasma FVIII concentration for a long time (on the 12^{th} to 20^{th} weeks, W12-W20). After the boosting administration on the 22^{nd} week (W22), the expression level rapidly returned to the level close to that before the decrease on W23 (the 23^{rd} week after the first injection, that is, the week immediately after the second injection). It shows that the second injection of the minicircle DNA provided in the present disclosure is still effective and will not produce a virus-specific immune response. It can be injected twice and restore or enhance the therapeutic effect by repeated administration as needed.

### Example 12: Long-term expression in vivo

Operations: Ten 6-8-week-old Balb/c mice were randomly divided into two groups: 5 in the MC.BDD-FVIII-F1 group and 5 in the MC.BDD-FVIII-L2 group. Blood was collected before the injection of minicircle DNA, and plasma was separated and stored at -80°C, which was used as a blank control sample (W0). Each mouse in the two groups was intramuscularly injected with the MC.BDD-FVIII-L1 minicircle DNA and the MC.BDD-FVIII-L2 minicircle DNA at a dose of 30 µg/50 µL/mouse (each mouse was injected with 50 µL containing 30 µg minicircle DNA), and then a pulsed electric field was applied (TERESA in vivo gene introduction instrument; parameters: 36 V, 10 ms, 1 Hz). On the 22^{nd} week after the injection, the mice were dosed by the same way. After the injection, blood was collected periodically, and plasma was separated and stored at -80°C. The target protein expression level in the plasma sample was detected using a Human FVIII ELISA kit (VisuLize Factor VIII PLUS Antigen ELISA Kit, Affinity Biologicals Inc).

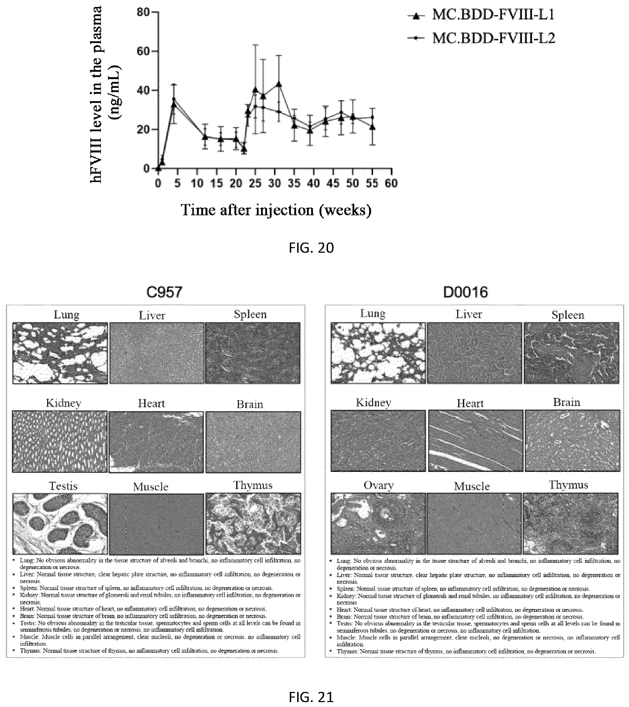

Results: As shown in FIG. 20, both MC.BDD-FVIII-L1 and MC.BDD-FVIII-L2 can be expressed in mice for a long time of 1 year or more, and the secondary injection of the minicircle DNA provided in the present disclosure is still effective for a long time, which will not produce a virus-specific immune response.

### Example 13: Safety Study

Operation: An adult female beagle dog with weight of 10-12 kg (No. D0016) and an adult male beagle dog with weight of 10-12 kg (No. C957) were injected with 1.2 mg of MC.BDD-FVIII-L1 minicircle DNA, respectively, and a pulsed electric field (36 V, 10 ms, 1 Hz) was applied. At the 6^{th} week after injection, samples were collected from lung, liver, spleen, kidney, heart, brain, ovary or testicle, muscle and thymus tissues, and subjected to sectioning, HE staining, and pathological examination. The results are shown in FIG. 21.

Results: 6 weeks after the injection of MC.BDD-FVIII-L1 minicircle DNA, the pathological examination performed on the lungs, liver, spleen, kidney, heart, brain, ovary (female dog D0016) or testis (male dog C957), muscle and thymus tissues of beagle dogs were all normal (FIG. 21). It shows that no obvious toxicity was found, and MC.BDD-FVIII-L1 has good safety.

The method of the present disclosure has been described through preferred embodiments. Relevant persons can obviously make modifications or appropriate changes and combinations to the methods and applications described herein within the content, spirit and scope of the present disclosure to implement and apply the technology of the present disclosure. Those skilled in the art can learn from the contents herein and appropriately improve the implementation of process parameters. It should be noted that all similar substitutions and modifications are obvious to those skilled in the art, and they are deemed to be included in the present disclosure.

## Claims

1. A linker peptide having an amino acid sequence of SEQ ID NO. 1 or SEQ ID NO. 2, or having an amino acid sequence having at least 80%-99% identity to any one of the nucleotide sequences or having at least a portion of either of the sequences.

2. A nucleotide sequence encoding the linker peptide of claim 1, wherein preferably, the nucleotide sequence comprises SEQ ID NO. 3 or SEQ ID NO. 4, or a codon-optimized sequence of either of the nucleotide sequences.

3. Use of the linker peptide of claim 1 or the nucleotide sequence of claim 2 for the construction of a recombinant FVIII protein or a variant thereof, or use of the nucleotide sequence of claim 2 for the construction of a nucleotide sequence of a recombinant FVIII protein or a variant thereof.

4. An FVIII protein or a variant thereof, wherein the linker peptide of the FVIII protein or the variant thereof is selected from the linker peptide of claim 1, or the nucleotide sequence of the linker peptide of the FVIII protein or the variant thereof is selected from the nucleotide sequence of claim 2.

5. An FVIII protein or a variant thereof having an amino acid sequence of SEQ ID NO. 5, SEQ ID NO. 6, SEQ ID NO. 21, SEQ ID NO. 23, SEQ ID NO. 43, SEQ ID NO. 44, SEQ ID NO. 50, SEQ ID NO. 51, SEQ ID NO. 52 or SEQ ID NO. 53, or having an amino acid sequence having at least 80%-99% identity to any one of the amino acid sequences or having at least a portion of any one of the amino acid sequences.

6. A nucleotide sequence encoding the FVIII protein or the variant thereof of either of claims 4-5, wherein preferably, the nucleotide sequence comprises SEQ ID NO. 7, SEQ ID NO. 8, SEQ ID NO. 22, SEQ ID NO. 24, SEQ ID NO. 38, SEQ ID NO. 39, SEQ ID NO. 54, SEQ ID NO. 56, SEQ ID NO. 58 or SEQ ID NO. 60, or a codon-optimized sequence of any one of the nucleotide sequences.

7. A recombinant gene vector, comprising a nucleotide sequence encoding the FVIII protein or the variant thereof of either of claims 4-5 or the nucleotide sequence of claim 6;

8. Preferably, the recombinant gene vector includes a non-viral vector or a viral vector; and/or

9. The non-viral vector is selected from a standard plasmid or other circular expression cassettes, or the viral vector is selected from a retroviral vector, a lentiviral vector, an adenoviral vector and an adeno-associated viral vector;

10. Preferably, the non-viral vector is selected from a minicircle DNA vector.

11. A parental plasmid for use in the production of a minicircle DNA, comprising a plasmid vector containing the nucleotide sequence of claim 6; or the parental plasmid is formed by inserting in a plasmid vector a promoter nucleotide sequence, an enhancer nucleotide sequence, a multiple cloning site nucleotide sequence, a polyA signal nucleotide sequence, and a DNA fragment of a gene of interest, wherein the DNA fragment of the gene of interest is located between the restriction endonuclease cleavage sites of the multiple cloning site, and the DNA fragment of the gene of interest comprises the nucleotide sequence of claim 6.

12. A method for preparing a minicircle DNA, comprising: transforming the parental plasmid of claim 8 into a host cell, performing induction to allow the parental plasmid to produce a minicircle DNA and a backbone DNA by site-specific recombination at specific recombination sites, and extracting the minicircle DNA using a plasmid DNA purification kit.

13. A minicircle DNA obtained according to the preparation method of claim 9, wherein preferably, the nucleotide sequence of the minicircle DNA is selected from SEQ ID NO. 25, SEQ ID NO. 26, SEQ ID NO. 27, SEQ ID NO. 28, SEQ ID NO. 46, SEQ ID NO. 47, SEQ ID NO. 49, SEQ ID NO. 55, SEQ ID NO. 57, SEQ ID NO. 59 or SEQ ID NO. 61.

14. A host cell, comprising a nucleotide sequence encoding the recombinant FVIII protein or the variant thereof of either of claims 4-5, the nucleotide sequence of claim 6, the recombinant gene vector of claim 7, or the minicircle DNA of claim 10, wherein preferably, the host cell includes a bacterial cell, a yeast cell, an insect cell or a mammalian cell.

15. A pharmaceutical composition, comprising the recombinant FVIII protein or the variant thereof of either of claims 4-5, the recombinant FVIII protein or the variant thereof encoded by the nucleotide sequence of claim 6, the recombinant gene vector of claim 7 or the minicircle DNA obtained according to the preparation method of claim 9 or the minicircle DNA of claim 10, and a pharmaceutically acceptable auxiliary material or carrier.

16. Use of the recombinant FVIII protein or the variant thereof of either of claims 4-5, the nucleotide sequence of claim 6, the recombinant gene vector of claim 7, the parental plasmid of claim 8, the minicircle DNA obtained according to the preparation method of claim 9 the minicircle DNA of claim 10, the host cell of claim 11 or the pharmaceutical composition of claim 12 for the preparation of a drug for treating a disease, wherein preferably, the disease is selected from a hereditary genetic defect disease; preferably, the disease is a disease caused by coagulation factor deficiency; preferably, the disease is a disease caused by a deficiency in FVIII; preferably, the disease is hemophilia.
